# EUROPEAN PATENT SPECIFICATION

(11) **EP 3 960 119 B1**
(45) Date of publication and mention of the grant of the patent: **07.08.2024**
(21) Application number: 21275115.0
(22) Date of filing: 24.08.2021
(51) Int. Cl.: A61C 7/22, A61C 7/12, A61C 7/00

(54) **DENTAL DEVICE**
ZAHNMEDIZINISCHE VORRICHTUNG
DISPOSITIF DENTAIRE

(30) Priority: 24.08.2020 GB 202013182; 13.01.2021 GB 202100436
(43) Date of publication of application: 02.03.2022
(73) Proprietor: Stainsby, Ryan, County Durham DL3 8RJ (GB)
(72) Inventor: Stainsby, Ryan, County Durham DL3 8RJ (GB)
(74) Representative: Elsworth, Dominic Stephen

(56) References cited:
- WO-A1-2015/164688
- US-A1- 2015 044 624
- US-A1- 2017 128 168

## Description

### Field of the Invention

The present invention relates to a dental device, in particular a device for providing and controlling movement of teeth; more particularly but not exclusively for orthodontists or similar specialists and their clients or device users.

### Background and Prior Art

Increasing awareness of cosmetic dentistry is a factor in many societies, leading to a proliferation of orthodontists and other specialists in these fields, as techniques and markets have improved.

Frequently in order to rearrange and straighten or correct teeth it can be necessary to wear braces and retainers and tracks, which act to gradually move the teeth over a long period of time to correct and improve their positions in a user's mouth.

This process is time-consuming and can additionally require the user to attend an orthodontist or specialist with frequent check-ups and adjustments made to the wires that are accomplishing the movement of the teeth.

This is resource inefficient both for the user and the orthodontist or specialist dentist.

The present invention arose in order to overcome problems suffered by existing devices.

US2017/128168 describes an orthodontic system including orthodontic brackets attached to surfaces of teeth and reaching an arch wire. A motor and gear system apply a force to the arch wire.

WO2015/164688 describes an electromechanical system for adjusting an orthodontic arch wire relative to orthodontic brackets attached to surfaces of teeth.

US2015/044624 describes an orthodontic device for correcting and changing tooth placement. The device may be computer controlled.

### Summary of the Invention

According to a first aspect of the invention there is provided a dental device as specified in Claim 1.

Preferably, the at least one elongate element is selected from the group comprising: a wire; a band; a chain; a coil spring and a ligature.

Preferably, the means for retracting or extending the at least one elongate element is selected from the group comprising: a linear actuator; a lever and a reel

The linear actuator may include a selected one of: a piston and cylinder; a rack and pinion; a worm drive; and contra-rotating rollers engaging the elongate element therebetween.

The means for retracting or extending the at least one elongate element may comprise a lever, the lever being arranged for rotation about an axis and the elongate element attached to the lever at a point distal from the axis.

Whilst a number of mechanisms for causing retraction and/or extension of the at least one elongate element have been described, the invention is not restricted to the described mechanisms. Any mechanism capable of causing retraction or extension of the at least one elongate element will suffice.

Advantageously, the at least one elongate element is arranged to exert a force on one or more teeth by either contacting one or more teeth or engaging with a member such as a pad or similar which may be attached to, onto or around one or more teeth. The member attached to said one more teeth may a selected one of: a pad; a bracket and a band.

In some embodiments the device comprises a plurality of wires, arranged to contact one or more teeth, so as to act to alter positioning of the tooth or teeth.

The at least one elongate element may be provided by a plurality of elongate elements.

In some embodiments the means for retracting and/or extending the at least one elongate element comprises a motor, which motor may be adapted to move the at least one elongate element, such as a wire or wires. Where the means for retracting and/or extending the at least one elongate element includes a reel the motor may drive or move the reel, for example shortening the wire(s) by turning the reel.

The means for retracting and/or extending the at least one elongate element may comprise a motor, the motor configured to power the means for retracting and/or extending the at least one elongate element.

The means for retracting and/or extending the at least one elongate element may be comprised in an elongate element movement module. Said module may be controlled by a local and/or remote control means. Such remote control means may be envisaged to be wireless, wherein the module may comprise a wireless transceiver.

The at least one elongate element may engage in an elongate element movement module.

The module may include a transceiver for wireless connection to a remote controller.

In some embodiments the wireless transceiver may be envisaged to operate with inter-machine operability such as Bluetooth (RTM). In other embodiments wireless connectivity may be provided by shortwave radio signals, infrared or other local or remote transmittable connectivity.

In some embodiments the module may comprise a socket, for example for connection of a wired connection, such as for example so as to allow data and/or electrical energy transmission.

The device may further comprise a source of electrical energy.

For example the device may comprise a battery, which may be rechargeable. In other embodiments, in addition or the alternative, the battery may be displaceable and/or disposable.

In some embodiments the device may comprise one or more sensors, which sensors may be envisaged to monitor conditions associated with the at least one elongate element and/or tooth or teeth. For example the module may comprise a sensor adapted to measure force acting on the at least one elongate element.

The device may comprise at least one sensor, the at least one sensor configured to monitor a condition associated with the at least one elongate element and/or tooth or teeth.

The at least one sensor may be configured to sense the force exerted on the at least one elongate element and/or tooth or teeth and/or the distance moved by the at least one elongate element and/or tooth or teeth.

The device may include a processor configured to control and/or recognise movement of the tooth or teeth and/or the at least one elongate element and the processor may be comprised in the module.

By providing means to control and/or recognise movement of the teeth and/or the at least one elongate element, for example by monitoring force acting on the at least one elongate element the orthodontist can monitor both the positioning of the teeth and the user's experience of the teeth movements, and may be enabled to both control and reflect the experience according to information acquired. For example, by monitoring how quickly the teeth move after adjustment of the at least one elongate element, the orthodontist may adjust the degree of adjustment of the at least one elongate element on each occasion of adjustment.

Orthodontists and users can then see the progress of treatment via software that may be installed on a plurality of different electronic items of equipment such as personal computers or smartphones.

This software may be programmed to generate indicia showing how far each tooth has moved and then the orthodontist can adapt as necessary. This advantageously means faster and more precise results for the patient.

In some embodiments the processor may comprise a microcontroller, which may include a timer, wherein the module may be configured to adjust the force acting on the at least one elongate element according to time of day, for example to increase force over a period of time incrementally and/or adjust for waking hours. Adjusting the force acting on the at least one elongate element may include a force release feature.

In some embodiments the micro-controller may be configured to control force acting on the at least one elongate element according to preferences that may be set by the user or orthodontist as appropriate. In other embodiments the user may be provided with an override option for use as required for example. An override feature may be useful during the period following adjustment of the at least one elongate element by the orthodontist. Users can experience moderate to severe pain in the days immediately following an adjustment (decrease in length and increase in force of the at least one elongate element). It would be useful to provide a means whereby the user may relax the adjustment for a period of time. A user may elect to relax tensioning in the at least one elongate element overnight, in order to be able to sleep for example, or on a more regular cyclic basis, in order to obtain pain relief. For example the micro controller could be programmed or otherwise caused to relax the force on the at least one elongate element for a period of 1 hour and apply the new force (associated with the new length of the at least one elongate element) for a period of 3 hours. This may be done over a period of a short number of days, for example four days. The duration of each part of the cycle may be changed in order to accustom the user to a new length of the at least one elongate element. For example, immediately after adjustment the period may be 1 hour at the new length/ force followed by 1 hour relaxed to the old force; followed by a period where the at least one elongate element is set to the new force for 1.5 hours, followed by 1 hour at the relaxed force; followed by 2 hours at the new force followed by 1 hour at the related force.

The force release and/or reduction means may be commanded by the processor.

The device may further comprise an elongate element lock, which may include an elongate element gripper.

In the above-mentioned scenarios, the relaxed force is not less than the force acting on the at least one elongate element immediately preceding the adjustment.

In some embodiments the device may include a clutch mechanism. Such a clutch mechanism may relieve the motor from force of the at least one elongate element, for example a gripper preventing return of the at least one elongate element until a solenoid valve release clutches for example.

In some embodiments the motor may be geared to increase torque and slow motion. It may be envisaged in use the device is maintained within a mouth. In this way it may be envisaged that the device may comprise a user attachment means. Such attachment means may comprise the wire or wires, or may comprise a separate attachment means. This attachment means may comprise adhesive for example.

The means for retracting and/or extending the at least one elongate element may comprise a manually operable mechanism, which may be comprised in the module. The module may include the means to adjust the length of the at least one elongate element manually. This may be desirable as a back up mechanism if the battery fails for example. The manual adjustment means may comprise a screw or pin engage able by a tool to generate rotation. Such rotation may drive the means for retracting or extending the at least one elongate element.

The module may be attached to one of the teeth. This may be achieved with a suitable adhesive or bands that fit around one or more teeth, typically to the rear of the user's mouth.

The module may be adapted for fixed attachment to a tooth or teeth.

In another embodiment the module is removable from the user's mouth, and the device includes a module mount attached to one or more of the user's teeth. The module is attached to the mount to affect a change in length of the at least one elongate element and is removed after the change has been effected. In such an arrangement, the mount includes a force maintaining means which holds the at least one elongate element in the position to which it was moved by the tensioner of the module. Such a force maintaining means may comprise a gripper arranged to engage with the at least one elongate element and which facilitates movement of the at least one elongate element in one direction (shortening of the at least one elongate element) and which resists movement in the opposite direction. The gripper may comprise a cam and/or a hinged element and/or teeth oriented in a particular direction; and/or an element that is deformable in one direction and which resists deformation in another direction.

The module may be adapted for removable attachment to a tooth or teeth, the tooth or teeth having a bracket fixed thereto, the module removably attachable to the bracket.

According to a second aspect of the present invention there is provided an apparatus for controlling movement of one or more teeth comprising a device substantially as described herein and a software application operated remotely.

Preferably, the software application is operated remotely.

The software application may monitor the device substantially continuously or periodically between adjustments of the length of the at least one elongate element.

### Brief Description of Drawings

In the Drawings, which illustrate preferred embodiments of a device of the invention and which are by way of example:

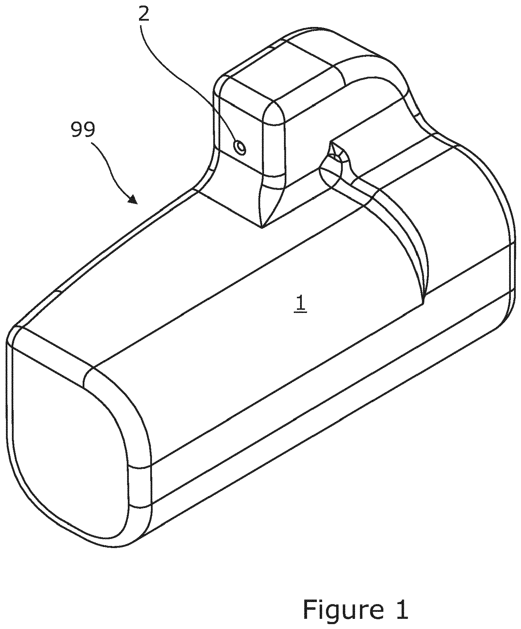

Figure 1 shows an isometric view of an embodiment of a module for the device according to the invention;

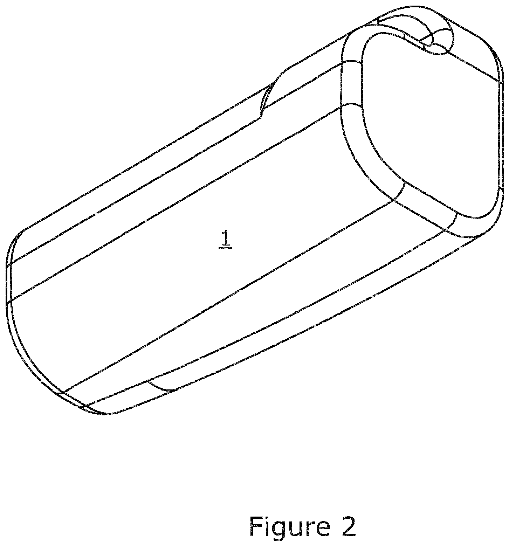

Figure 2 shows a reverse isometric view of the embodiment of the module shown in Figure 1;

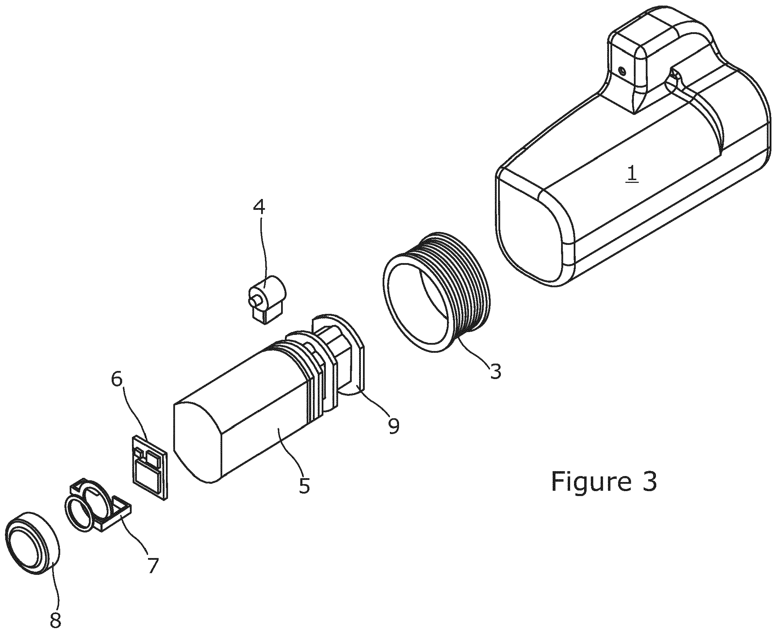

Figure 3 shows an exploded isometric view of the embodiment of the module shown in Figure 1;

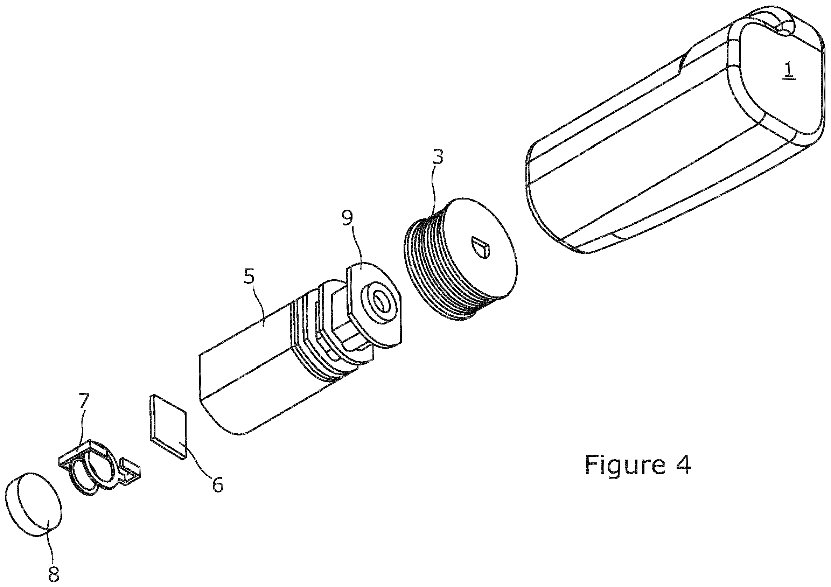

Figure 4 shows a reverse exploded isometric view of the embodiment of the module shown in Figure 1;
Figure 5 shows a diagrammatic sketch of a view of the embodiment of the device in use on a mouth, with a wire;
Figure 6 illustrates an embodiment of the invention where the module is removable from the user's mouth;
Figures 7a to 7e illustrate alternative mechanisms for retracting or extending the elongate element of the device of the invention;
Figure 8 is a block diagram illustrating the electronic system of the module;
Figure 9 illustrates a smart phone programmed with the software of the invention;
Figure 10 illustrates a computer device operable by an orthodontist; and
Figure 11 illustrates a computer device operable by a patient.

### Detailed Description of Figures

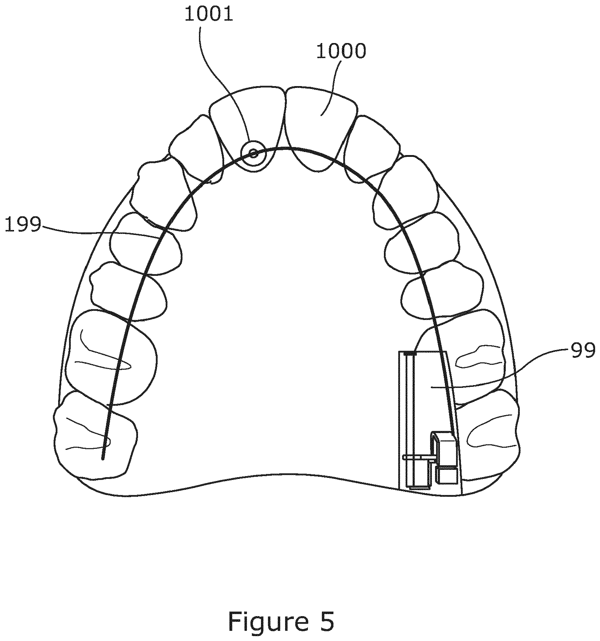

With reference to the figures there is shown an embodiment of the device 99 for apparatus generally comprising one or more wires 199 in use arranged to contact and exert force on one or more teeth 1000, the wire being attached to the teeth 1000 by pads 1001 attached to each tooth (only one pad is illustrated in Figure 5 for the sake of clarity) and a reel 3, which reel is arranged to move the wire 199.

The device comprises an internal battery for powering a motor and a transceiver is provided to receive a wireless signal which is used to control the motor.

The illustrated embodiment of the device comprises a monolithic housing 1 in which are situated components configured to adjust and hold force on the wire 199 and hence adjust the position of teeth 1000. It is envisaged that housing 1 is mounted inside the patient's mouth in use, and which allows an orthodontist or specialist (referred to hereafter as orthodontist) and patient or user (referred to hereafter as patient) to adjust the force of braces via a software application.

At the instance of first fitting of the device 99, associated wire 199 and pads 1001 the orthodontist develops a treatment plan. Without the device of the invention, the orthodontist would require the patient to be examined in person periodically. Upon any particular examination the orthodontist would decide whether to apply more force to the wire 199 and hence progress the treatment. With the benefit of the invention, the orthodontist sets up a programme of adjustment to the wire 199 that is delivered by the device to a great extent. The orthodontist can monitor the force on the wire 199 at any time. The software may be programmed to make a series of adjustments. For example, the software may be programmed to exert more force on the wire 199 as soon as a teeth movement criteria has been satisfied. Alternatively, the software may be programmed to alert the orthodontist to a teeth movement criteria being satisfied. The orthodontist may then issue a command via the software to cause the force acting on the wire 199 to be increased.

The device includes electronic hardware and the software measures and records progress of movement of the teeth 1000. The gathered information may be used to modify the treatment plan created by the orthodontist, making the process easier, controlled and time effective, both from a patient and orthodontist's perspective.

The illustrated embodiment comprises the following:
Housing 1, in stainless steel, gold-plated. In some embodiments the housing may comprise a plastic area to let the signal of the transceiver out from the module.
Injection moulded reel 3 in Nylon (RTM)
Motor 5
Gearbox 9
Brace wire 199 in stainless steel
PCB control processor, UNITFR4 board 6
CR927 battery 8
Battery clip 7

The gold-plated stainless steel of the housing 1 is a non-reactive material that will protect the components inside from acids in food and drink that the patient consumes.

The device in use is mounted securely at the back of the patient's mouth and connects with an internal brace wire 199 that is mounted onto the teeth 1000 typically by means of pads 1001. The device is designed to be non-intrusive and made from a non-reactive material.

The reel 3 is rotatable by the motor to apply force to the wire 199 according to an instruction from at the PCB 6, from the orthodontist or patient.

The battery 8 provides power to the circuit board and the motor. The battery is envisaged to be sized such that it will hold sufficient charge to last the duration of the treatment.

The PCB 6 includes a Bluetooth (RTM) transceiver which allows the brace force to be controlled via a software application on a phone or tablet.

The gearbox 9 allows the small motor 5 to apply an amount of force to the brace wire 199 sufficiently large to cause movement of the teeth 1000.

The wire 199 exits the housing at a small exit 2 and a wire gripper 4 provides a lock which prevents the wire from loosening over time and also relieves the motor 5 and gearbox 9 from constant force. The wire gripper 4 may be a cam in some embodiments.

A solenoid may be provided to allow the force of the brace to be released if there is too much discomfort, the solenoid operable to release the wire gripper 4.

The treatment can be started and controlled via the application by the patient and/or the orthodontist after fitting by the orthodontist.

In use:
The specialist takes a precise 3D digital scan of the patient or user's teeth and records the data.

The data is recorded onto the application which provides for the formulation of a treatment plan. Typically, this will be a treatment plan that is the most time efficient and/or the best way of achieving the desired result. It may be envisaged that the method of adjusting the position of teeth 1000 enabled by the device may allow for greater computational accuracy, through accumulation of data for example acquired through a plurality of such devices in use.

The microprocessor is operative to control the motor and at least one sensor senses a characteristic associated with the at least one wire such as sense force, and is operative to send a signal to the microprocessor.

In this way the present invention also provides a system for controlling movement of one or more teeth comprising a device according to any claim and a remote control device which includes a processor operated in accordance with software.

The specialist synchronises the device to the application via Bluetooth (RTM).

The housing 1 of the device 99 is then mounted to the back corner of the user's mouth with the wire 199 fixed to the teeth. The device is configured to be mounted or fixed to teeth or gums with glue or cement in a manner similar to braces, conditioning the enamel before bonding to increase effectiveness of attachment, where pretreatment causes microscopic changes in the enamel, allowing bond materials such as cement or adhesive to flow into the enamel, giving a micro-mechanical attachment.

In some embodiments it may be envisaged that the housing 1 may be mounted using rings or bands to a tooth or teeth. In still further embodiments the housing 1 may be screwed into teeth.

Using the application to start the treatment or after any adjustment in force acting on the wire 199, the orthodontist or patient may override manually the amount of force applied to the wire 199 if there is too much discomfort or pain.

In some embodiments the wire may be envisaged to be attached onto or around teeth or other items, for example which items may be envisaged to be placed against the teeth or active in relation to the positions of the teeth, so as to push or pull against the teeth. In other embodiments the wire may be sprung or biased so as to push the teeth.

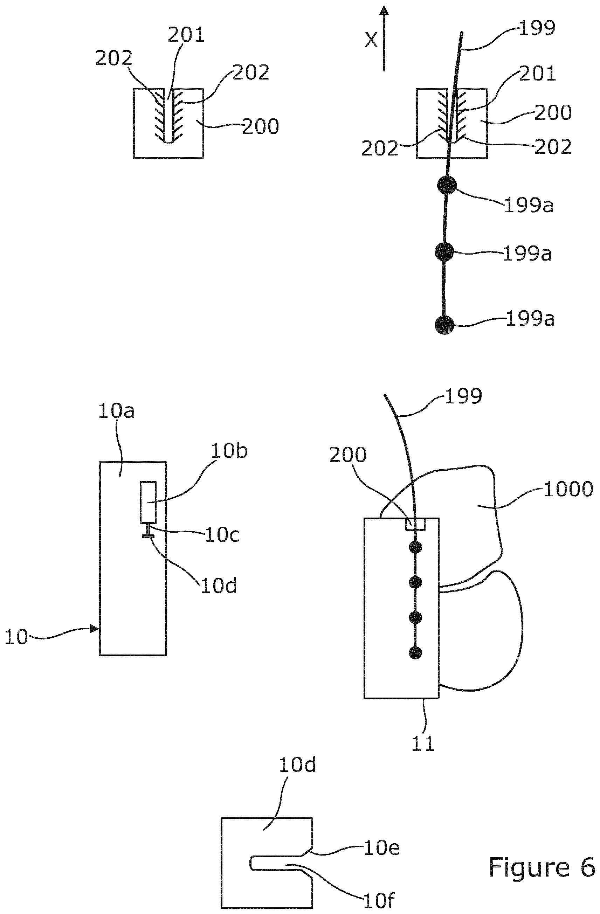

Referring now to Figure 6, an alternative arrangement is illustrated in which the module 10 is detachably mountable on the teeth 1000 of the user. A mount 11 is attached to the teeth, two in the illustrated example. Typically, the mount would be attached to the teeth 1000 with adhesive or dental cement. The mount 11 remains in place throughout the duration of the treatment. The mount 11 and the module 10 are provided with co-operating parts which allow the module 10 to be attached securely to the mount 11.

The wire 199 is provided with a number of protrusions 199a. When the module 10 is attached to the mount 11, a plate 10d engages with the wire 199, the wire being received in the slot 10f. The plate 10d includes a mouth 10e which guides the wire 199 into the slot 10f as the module 10 is presented up to the mount 11. The plate 10d is attached to the piston 10c of a linear actuator which comprises the piston 10c and a cylinder 10b, the linear actuator being mounted in a body 10a of the module 10. When the piston 10c is extended, the plate 10d engages with one of the protrusions 199a to retract the wire 199. As can be seen from Figure 6, a non-return mechanism is provided. The non-return mechanism comprises a block 200 with a trough 201 extending therethrough. The side walls of the trough 201 are provided with teeth 202 which are angled so as to resist movement of the wire 199 in the direction X, but permit movement in the direction opposite to direction X. By providing a trough 201, the wire 199 can be engaged therein with the protrusions 199a to one side of the block 200 as shown in Figure 6. The function of the protrusions is to provide for engagement with the plate 10d of the linear actuator. When the wire 199 is retracted to move the teeth, the wire is held in the new retracted configuration by the teeth 202.

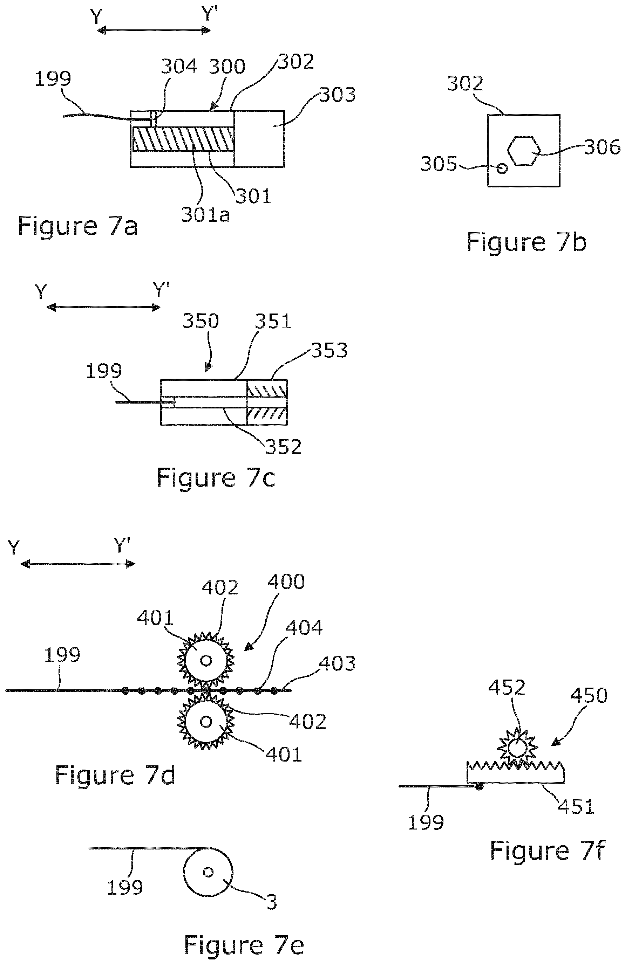

Referring now to Figures 7a to 7f, a number of alternative linear mechanisms for retracting or extending the wire 199 illustrated in Figure 5. Each generates movement in a substantially linear direction.

Figure 7a illustrates a worm drive 300 comprising a worm shaft 301 which is threaded externally with threads 301a. The worm 301 is mounted in a housing 302 which also mounts a motor 303 to which the worm shaft 301 is attached. A worm follower 304 is also mounted in the housing and engages with threads 301a. The wire 199 is connected to the worm follower 304. The worm follower 304 moves in the direction Y or Y' depending on the direction of rotation of motor 303. As can be seen from Figure 7b, the wire 199 passes through a hole 305 in the housing 302. Also shown in Figure 7b is a manual adjuster in the form of a key 306. The manual adjuster 306 illustrated in this embodiment is operable by a suitably sized Allen key and provides a back up in the event of failure of the motor 303 or the battery powering the motor 303 for example.

Figure 7c illustrates a linear actuator 350 comprising a housing 351 in which a threaded rod 351 is mounted. A motor 353 is attached to the housing 351. The threaded rod 352 engages with a correspondingly threaded sleeve extending through the centre of the motor 353 so that when the motor 353 is rotated the threaded rod 351, to which the wire 199 is connected, moves back or forth in the direction Y or Y'.

Figure 7d illustrates a mechanism for retracting or extending the wire 199 which comprises two rollers 401, each of which has circumferentially mounted teeth 402. The wire 199 is attached to a chain 403 which comprises links 404. When the rollers 401 are caused to rotate, each roller being driven to rotate in a direction opposite to the other, the teeth 402 engage with the links 404 of the chain 403 to generate movement of the wire in the direction Y or Y'.

Figure 7e illustrates a reel 3, the wire 199 being wound around the reel 3. Rotation of the reel 3 generates movement of the wire 199 in the direction Y or Y', depending on the direction of rotation of the reel.

Figure 7f illustrates a rack and pinion actuator 450 comprising a rack 451 to which the elongate element 199 is attached and a pinion 452 arranged to mesh with the rack 451. The pinion may be driven by a motor (not shown).

The mechanisms illustrated in Figures 7a to 7f are by way of example and are not limitative. Any mechanism capable of adjusting the force on an elongate element and capable of fitting in the space limitations of the users's mouth could be used.

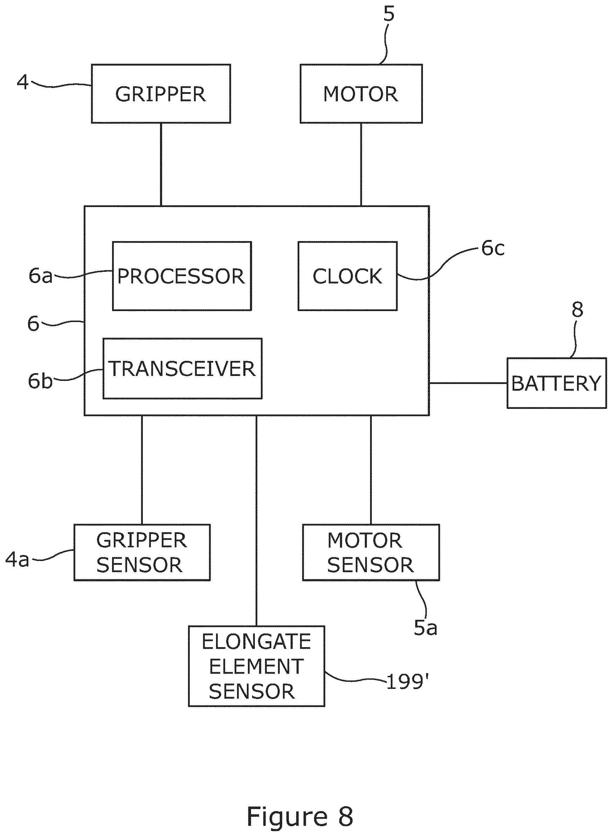

Referring now to Figure 8, a micro controller is provided by the printed circuit board 6 which includes a processor 6a, a transceiver 6b and a clock 6c. Connected to the printed circuit board 6 are a number of sensor inputs and a number of signal outputs as well as the battery 8 which powers both the components of the printed circuit board 6 an other components requiring electrical power, such as the motor 5.

In normal operation, a command signal is received by the transceiver 6b. The command signal may be to shorten the elongate element by 0.5 mm. The processor sends a signal commanding the motor 5 to turn. When the elongate element sensor 199' senses that the elongate element has in fact been shortened by 0.5 mm, the elongate element sensor 199' sends a signal to the processor 6a indicating that the elongate element has been shortened by the desired amount. The processor 6a is programmed with logic causing the signal commanding the motor 5 to turn to be switched to a signal commanding the motor 5 to stop.

When the motor is turning, the motor sensor 5a provides a signal to the processor 6a indicating that the motor is turning, and when the motor ceases to turn, the motor sensor 5a provides a signal indicating that the motor 5 is not turning.

The micro-controller illustrated in Figure 8 is configured to work with the optional elongate element gripper 4. As described above, the function of the gripper 4 is to hold the elongate element in a particular setting and to relieve the force that would otherwise act on the motor 5.

It is preferred that the gripper 4 grips the elongate element unless a positive force to release the gripper is applied. The processor 6a is programmed with logic such that when the command signal to shorten the elongate element 199 is received by the transceiver 6b, the processor 6a sends a signal to a solenoid associated with the gripper 4 which energises the solenoid to release the grip of the gripper 4 on the elongate element 199. The processor 6a is programmed such that when the motor sensor 5a sends a signal to the processor 6a indicating that the motor 5 is turning, the signal received by the solenoid associated with the gripper 4 remains energised and keeps the gripper off the elongate element 199. When the elongate element 199 has been shortened by the desired amount, the motor stops turning, the motor sensor 5a indicates this. The processor 6a is programmed with logic that causes the solenoid associated with the gripper 4 to be de-energised and the gripper 4 grips the elongate element 199.

The elongate element sensor 5a senses the position and distance moved by the elongate element 199. The signal to shorten the elongate element 199 is received by the transceiver 6b and the signals discussed above are sent, thereby allowing shortening of the elongate element. The sensor 199' monitors the length of the elongate element as it is being adjusted. When the new length has been reached, the sensors 199' sends a signal indicating the same. The processor 6a is programmed with logic which causes the motor to stop turning when the elongate element sensor 199' issues a signal indicating that the new length has been reached.

The clock 6c allows the time period of a whole treatment to be monitored, and also facilitates a set back function. If a set back function is provided, one example of how such function may work would be as follows:
A new length for the elongate element 199 is set. This may cause pain for the patient. The set back function may allow the elongate element to return to its length immediately prior to adjustment. The patient or the orthodontist may set the time period for set back to eight hours, daily for three days following shortening of the elongate element 199. Typically, this would coincide with the time when the patient usually sleeps.

The signal received by the processor 6a via the transceiver would be to release the gripper 4. This in itself may be sufficient to provide the pain relief sought by the patient. Alternatively, and in addition, a signal may be sent to the motor causing the motor to reverse, returning the elongate element 199 to its length immediately prior to shortening. Either the processor 6a is programmed with logic, or a signal is received by the transceiver, such that at the end of the first set back period, measured by the clock 6c, the elongate element 199 is shortened once again as described above. The set back period may be repeated for a number of days.

Instead of adjusting and monitoring the length of the elongate element, the force exerted on the elongate element may be monitored and adjusted.

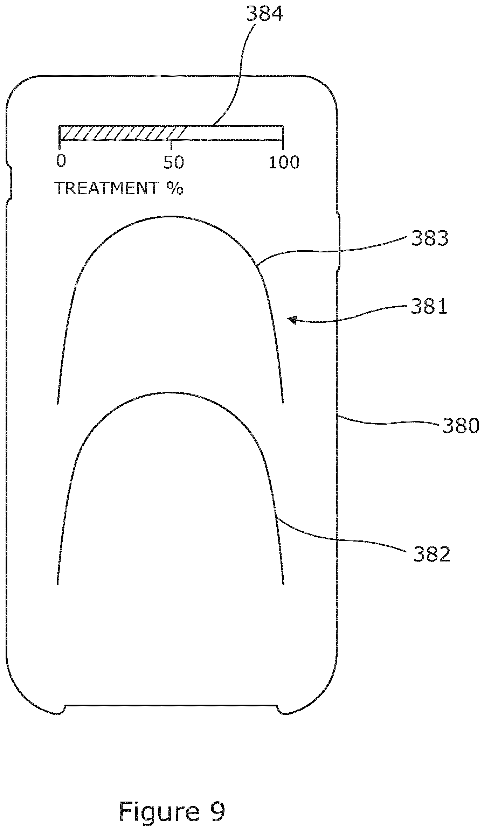

Figure 9 is an illustration of the imagery that may be displayed on the screen of a computer device, such as a smart phone 380 or other computer, programmed with the software of the invention. The smart phone 380 comprises a display screen 381. An image of the starting shape of the teeth before treatment is shown at 382. The target shape 383 is also shown on the display screen 381 above the starting shape 382. At the top of the display screen 381 is a bar 384 which illustrates in percentage terms how far the treatment has progressed. In the illustration treatment has progressed just beyond 50%.

The images presented on the display screen 381 may depict the starting and end points of a whole treatment programme, or the starting and end points of steps in a programme.

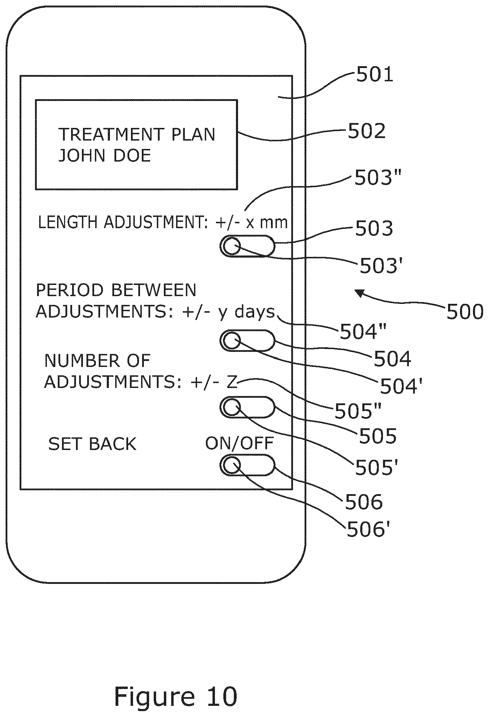

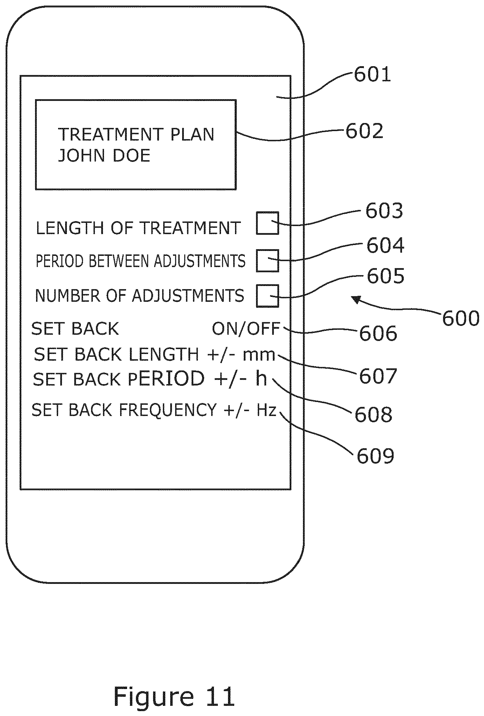

Figures 10 and 11 also illustrate a computer device in the form of a smart phone for use by the orthodontist and patient respectively. Referring first to Figure 10, the smart phone 500 is programmed with the software application which allows the orthodontist to set the parameters of a treatment programme. The parameters that the orthodontist may set are: the length of adjustment, that is the distance the elongate element is moved by at each adjustment; the period between adjustment, that is the number of days between any two adjustments; and the number of adjustments.

As can be seen from the Figure, the smart phone comprises a screen 501 provides various data input possibilities. The name of the patient can be entered in the box 502. The box 503 allows the length of each adjustment to be selected. By touching the button 503' and moving his finger to the right the orthodontist switches the length adjustment on. The orthodontist may then increase or decrease the length x mm of adjustment using the +/- function 503".

The period (y days) between adjustments is set by the orthodontist first touching the button 504' with his finger and swiping to the right. The orthodontist may then increase or decrease the period between adjustments using the +/- function 504".

The number of adjustments (Z) is set in a similar manner. The orthodontist touches the button 505' and swipes to the right. The number of adjustments is set using the +/- function 505".

A set back function is also provided. This allows the patient to relax the force on the elongate element. By swiping the button 506' in the box 506 to the right the setback function is switched off.

Figure 11 illustrates the user's smart phone 600. The smart phone 600 includes a screen which provides indications of the patient receiving treatment in box 602, the length of treatment in box 603, the period between treatments in box 604, the tube rod adjustments in box 605 and a set back on/off function with on/off switch 606. For the user to be able to switch the set back function on, the orthodontist must have switched the set back function on in the treatment plan.

When switched to 'on' the set back function allows the patient to select the distance by which the elongate element is relaxed with +/- function 607, the set back period with +/- function 608 and the set back frequency with +/- function 609. The software application may be programmed with maximums for each set back parameter. For example, the set back length may be set such that the elongate element cannot be relaxed to a length that is greater than the length prior to the current adjustment. The set back period may be set to a maximum of a part of a day, for example 12 hours. Typically, the frequency of the set back would be daily, for example overnight, for a few days after an adjustment. The patient switches the set back function off when he no longer feels the need to relax the elongate element. The set back frequency should not be short and repetitive as that would loosen the tooth/teeth.

The orthodontist and user my be able to switch between the screens shown in Figures 8, 10 and 11 respectively. The software application may also provide a home screen (not shown).

The invention has been described by way of examples only and it will be appreciated that variation may be made to the above-mentioned embodiments without departing from the scope of invention as defined by the claims, in particular but not solely combination of features of described embodiments.

## Claims

1. A dental device (99) for location in a user's mouth comprising at least one elongate element (199), the at least one elongate element (199) arranged to exert a force on one or more teeth (1000) and a means for retracting and/or extending the at least one elongate element (199) in order to manipulate the one or more teeth (1000), and wherein the means for retracting and/or extending the at least one elongate element (199) comprises a motor (5), the motor (5) configured to power the means for retracting and/or extending the at least one elongate element (199), and wherein the device includes a processor (6) configured to control and/or recognise movement of the tooth or teeth (1000) and/or the at least one elongate element (199); and wherein the device further includes a force release and/or force reduction means, and wherein the processor (6a) includes a timer, and wherein the module (10) is configurable to reduce the force acting on the at least one elongate element from a prescribed force for a period of time, and to return the force acting on the at least one elongate element (199) to the prescribed force at the end of the period of time.

2. A dental device (99) according to Claim 1, wherein the at least one elongate element (199) is selected from the group comprising: a wire; a band; a chain; a coil spring and a ligature.

3. A dental device (99) according to Claim 1 or 2, wherein: the means for retracting or extending the at least one elongate element (199) is selected from the group comprising: a linear actuator; a lever and a reel (3); or the means for retracting or extending the at least one elongate element (199) is a linear actuator and the linear actuator is a selected one of: a piston (10c) and cylinder (10b); a rack and pinion (450); a worm drive (300); and contra-rotating rollers engaging the elongate element (199) therebetween.

4. A dental device (99) according to any preceding claim, wherein: the at least one elongate element (199) is arranged to exert a force on one or more teeth (1000) by either contacting one or more teeth (1000) or engaging with a member attached to said one or more teeth (1000); or the at least one elongate element (199) is arranged to exert a force on one or more teeth (1000) by either contacting one or more teeth or engaging with a member attached to said one or more teeth (1000) and the member attached to said one more teeth is a selected one of: a pad; a bracket and a band.

5. A dental device (99) according to any preceding claim, wherein: the at least one elongate element (199) engages in an elongate element movement module (10); or the at least one elongate element (199) engages in an elongate element (199) movement module (10) and the module (10) includes a transceiver for wireless connection to a remote controller; and/or the module (10) comprises a socket, the socket providing for a wired connection; or the at least one elongate element (199) engages in an elongate element movement module (10) and the module (10) is adapted for fixed attachment to a tooth or teeth (1000); or the at least one elongate element (199) engages in an elongate element movement module (10) and the module (10) is adapted for removable attachment to a tooth or teeth (1000), the tooth or teeth (1000) having a bracket fixed thereto, the module (10) removably attachable to the bracket.

6. A dental device (99) according to any preceding claim, further comprising a source of electrical energy.

7. A dental device (99) according to any preceding claim, wherein: the device (99) comprises at least one sensor, the at least one sensor configured to monitor a condition associated with the at least one elongate element (199) and/or tooth or teeth (1000); or the device comprises at least one sensor, the at least one sensor configured to monitor a condition associated with the at least one elongate element (199) and/or tooth or teeth (1000) and the at least one sensor is configured to sense the force exerted on the at least one elongate element (199) and/or tooth or teeth (1000) and/or the distance moved by the at least one elongate element (199) and/or tooth or teeth (1000).

8. A dental device (99) according to any preceding claim, wherein: the processor is comprised in a micro-controller.

9. A dental device (99) according to any preceding claim, wherein the force release and/or reduction means are commanded by the processor (6).

10. A dental device (99) according to any preceding claim, further comprising: an elongate element lock; or an elongate element lock, wherein the elongate element lock includes an elongate element gripper (4).

11. A dental device (99) according to any preceding claim, wherein the motor (5) is provided with a clutch mechanism.

12. Apparatus for controlling movement of one or more teeth comprising a dental device (99) as claimed in any of Claims 1 to 11, a computer device programmed with a software application and signal transmission means providing for the transmission of signals between the computer device and the dental device.

## Patentansprüche

1. Zahnmedizinische Vorrichtung (99) zur Anordnung in einem Mund eines Benutzers, umfassend mindestens ein längliches Element (199), wobei das mindestens eine längliche Element (199) dazu eingerichtet ist, eine Kraft auf einen oder mehrere Zähne (1000) auszuüben, und ein Mittel zum Einfahren und/oder Ausfahren des mindestens einen länglichen Elements (199), um den einen oder die mehreren Zähne (1000) zu manipulieren, und wobei das Mittel zum Einfahren und/oder Ausfahren des mindestens einen Elements (199) einen Motor (5) umfasst, wobei der Motor (5) dazu konfiguriert ist, das Mittel zum Einfahren und/oder Ausfahren des mindestens einen länglichen Elements (199) anzutreiben, und wobei die Vorrichtung einen Prozessor (6) beinhaltet, der dazu konfiguriert ist, eine Bewegung des Zahns oder der Zähne (1000) und/oder des mindestens einen länglichen Elements (199) zu kontrollieren und/oder zu erkennen; und wobei die Vorrichtung weiterhin ein Kraftfreigabe- und/oder Kraftverringerungsmittel beinhaltet, und wobei der Prozessor (6a) einen Zeitgeber beinhaltet, und wobei das Modul (10) dazu konfigurierbar ist, die Kraft, die auf das mindestens eine längliche Element einwirkt, für einen Zeitraum von einer vorgeschriebenen Kraft zu verringern und die Kraft, die auf das mindestens eine längliche Element (199) einwirkt, zum Ende des Zeitraums auf die vorgeschriebene Kraft zurückzusetzen.

2. Zahnmedizinische Vorrichtung (99) nach Anspruch 1, wobei das mindestens eine längliche Element (199) ausgewählt ist aus der Gruppe umfassend: einem Draht; einem Band; einer Kette; einer Spiralfeder und einer Abbindungsschnur.

3. Zahnmedizinische Vorrichtung (99) nach Anspruch 1 oder 2, wobei: das Mittel zum Einfahren oder Ausfahren des mindestens einen länglichen Elements (199) ausgewählt ist aus der Gruppe umfassend: einem Linearaktor; einem Hebel und einer Spule (3); oder das Mittel zum Einfahren oder Ausfahren des mindestens einen länglichen Elements (199) ein Linearaktor ist und der Linearaktor ein ausgewählter ist aus: einem Kolben (10c) und einem Zylinder (10b); einer Zahnstange (450; einem Schneckengetriebe (300) und sich gegenläufig drehenden Walzen, die das längliche Element (199) dazwischen in Eingriff nehmen.

4. Zahnmedizinische Vorrichtung (99) nach einem vorhergehenden Anspruch, wobei: das mindestens eine längliche Element (199) dazu eingerichtet ist, eine Kraft auf einen oder mehrere Zähne (1000) durch entweder Berühren eines oder mehrerer Zähne (1000) oder Ineingriffkommen mit einem Teil, das an dem einen oder den mehreren Zähnen (1000) angebracht ist, auszuüben; oder das mindestens eine längliche Element (199) dazu eingerichtet ist, eine Kraft auf einen oder mehrere Zähne (1000) durch entweder Berühren eines oder mehrerer Zähne oder Ineingriffkommen mit einem Teil, das an dem einen oder den mehreren Zähnen (1000) angebracht ist, auszuüben, und wobei das Teil, das an dem einen oder den mehreren Zähnen angebracht ist, ein ausgewähltes ist aus: einer Kompresse; einer Klammer und einem Band.

5. Zahnmedizinische Vorrichtung (99) nach einem vorhergehenden Anspruch, wobei: das mindestens eine längliche Element (199) in einem Modul (10) zur Bewegung eines länglichen Elements in Eingriff kommt; oder das mindestens eine längliche Element (199) in einem Modul (10) zur Bewegung eines länglichen Elements (199) in Eingriff kommt und das Modul (10) einen Transceiver zur drahtlosen Verbindung mit einer Fernbedienung beinhaltet; und/oder das Modul (10) eine Anschlussdose umfasst, wobei die Anschlussdose eine drahtgebundene Verbindung bereitstellt; oder das mindestens eine längliche Element (199) in einem Modul (10) zur Bewegung eines länglichen Elements in Eingriff kommt und das Modul (10) zum festen Anbringen an einem Zahn oder Zähnen (1000) geeignet ist; oder das mindestens eine längliche Element (199) in einem Modul (10) zur Bewegung eines länglichen Elements in Eingriff kommt und das Modul (10) zum lösbaren Anbringen an einem Zahn oder Zähnen (1000) geeignet ist, wobei der Zahn oder die Zähne (1000) eine daran befestigte Klammer aufweisen, wobei das Modul (10) lösbar an der Klammer anbringbar ist.

6. Zahnmedizinische Vorrichtung (99) nach einem vorhergehenden Anspruch, weiterhin umfassend eine Quelle von elektrischer Energie.

7. Zahnmedizinische Vorrichtung (99) nach einem vorhergehenden Anspruch, wobei: die Vorrichtung (99) mindestens einen Sensor umfasst, wobei der mindestens eine Sensor dazu konfiguriert ist, einen Zustand, der mit dem mindestens einen länglichen Element (199) und/ oder dem Zahn oder den Zähnen (1000) assoziiert ist, zu überwachen; oder die Vorrichtung mindestens einen Sensor umfasst, wobei der mindestens eine Sensor dazu konfiguriert ist, einen Zustand, der mit dem mindestens einen länglichen Element (199) und/oder dem Zahn oder den Zähnen (1000) assoziiert ist, zu überwachen, und der mindestens eine Sensor dazu konfiguriert ist, die Kraft, die auf das mindestens eine längliche Element (199) und/oder den Zahn oder die Zähne (1000) ausgeübt wird, und/oder die Strecke, die das mindestens eine längliche Element (199) und/oder der Zahn oder die Zähne (1000) sich bewegt hat bzw. haben, zu erfassen.

8. Zahnmedizinische Vorrichtung (99) nach einem vorhergehenden Anspruch, wobei: der Prozessor in einem Mikrocontroller enthalten ist.

9. Zahnmedizinische Vorrichtung (99) nach einem vorhergehenden Anspruch, wobei die Kraftfreigabe- und/oder -verringerungsmittel von dem Prozessor (6) gesteuert werden.

10. Zahnmedizinische Vorrichtung (99) nach einem vorhergehenden Anspruch, weiterhin umfassend: eine Sperre für ein längliches Element; oder eine Sperre für ein längliches Element, wobei die Sperre für ein längliches Element einen Greifer (4) für ein längliches Element beinhaltet.

11. Zahnmedizinische Vorrichtung (99) nach einem vorhergehenden Anspruch, wobei der Motor (5) mit einem Kupplungsmechanismus versehen ist.

12. Vorrichtung zur Kontrolle einer Bewegung eines oder mehrerer Zähne, umfassend eine zahnmedizinische Vorrichtung (99) nach einem der Ansprüche 1 bis 11, eine Computervorrichtung, die mit einer **Softwareanwendung programmiert ist, und ein** Signalübertragungsmittel, das die Übertragung von Signalen zwischen der Computervorrichtung und der zahnmedizinischen Vorrichtung bereitstellt.

## Revendications

1. Un dispositif dentaire (99) destiné à être placé dans la bouche d'un utilisateur comprenant un ou plusieurs éléments allongés (199), le ou les éléments allongés (199) étant agencés pour exercer une force sur une ou plusieurs dents (1000) et un moyen de rétraction et/ou d'extension du ou des éléments allongés (199) afin de manipuler la ou les dents (1000), et dans lequel le moyen de rétraction et/ou d'extension du ou des éléments allongés (199) comprend un moteur (5), le moteur (5) étant configuré pour propulser le moyen de rétraction et/ou d'extension du ou des éléments allongés (199), et dans lequel le dispositif inclut un processeur (6) configuré pour commander et/ou reconnaître un déplacement de la dent ou des dents (1000) et/ou du ou des éléments allongés (199) ; et dans lequel le dispositif inclut en outre un moyen de réduction de force et/ou de libération de force, et dans lequel le processeur (6a) inclut une minuterie, et dans lequel le module (10) est configurable pour réduire la force agissant sur le ou les éléments allongés à partir d'une force prescrite pendant une période de temps, et pour renvoyer la force agissant sur le ou les éléments allongés (199) à la force prescrite à la fin de la période de temps.

2. Un dispositif dentaire (99) selon la revendication 1, dans lequel le ou les éléments allongés (199) sont sélectionnés dans le groupe comprenant : un fil métallique ; une bande ; une chaîne ; un ressort hélicoïdal et une ligature.

3. Un dispositif dentaire (99) selon la revendication 1 ou 2, dans lequel : le moyen de rétraction ou d'extension du ou des éléments allongés (199) est sélectionné dans le groupe comprenant : un actionneur linéaire ; un levier et une bobine (3) ; ou le moyen de rétraction ou d'extension du ou des éléments allongés (199) est un actionneur linéaire et l'actionneur linéaire est sélectionné parmi : un piston (10c) et un cylindre (10b) ; une crémaillère (450) ; une vis sans fin (300) ; et des rouleaux contrarotatifs mettant en prise les éléments allongés (199) entre eux.

4. Un dispositif dentaire (99) selon l'une quelconque des revendications précédentes, dans lequel : le ou les éléments allongés (199) sont agencés pour exercer une force sur une ou plusieurs dents (1000) soit en entrant en contact avec une ou plusieurs dents (1000), soit en se mettant en prise avec un élément fixé à ladite ou lesdites dents (1000) ; ou bien le ou les éléments allongés (199) sont agencés pour exercer une force sur une ou plusieurs dents (1000), soit en entrant en contact avec une ou plusieurs dents, soit en se mettant en prise avec un élément fixé à ladite ou lesdites dents (1000) et l'élément fixé à ladite ou lesdites dents est sélectionné parmi : un tampon ; un patte d'attache et une bande.

5. Un dispositif dentaire (99) selon l'une quelconque des revendications précédentes, dans lequel : le ou les éléments allongés (199) se mettent en prise dans un module de déplacement d'éléments allongés (10) ; ou le ou les éléments allongés (199) se mettent en prise dans un module de déplacement (10) des éléments allongés (199) et le module (10) inclut un émetteur-récepteur pour une connexion sans fil à une télécommande ; et/ou le module (10) comprend une prise de courant, la prise de courant permettant une connexion filaire ; ou le ou les éléments allongés (199) se mettent en prise dans un module de déplacement d'éléments allongés (10) et le module (10) est adapté pour permettre une fixation fixe à la dent ou aux dents (1000) ; ou le ou les éléments allongés (199) se mettent en prise dans un module de déplacement d'éléments allongés (10) et le module (10) est adapté pour se fixer de manière amovible à la dent ou aux dents (1000), la dent ou les dents (1000) ayant une patte d'attache y étant fixée, le module (10) pouvant être fixé de manière amovible à la patte d'attache.

6. Un dispositif dentaire (99) selon l'une quelconque des revendications précédentes, comprenant en outre une source d'énergie électrique.

7. Un dispositif dentaire (99) selon l'une quelconque des revendications précédentes, dans lequel : le dispositif (99) comprend un ou plusieurs capteurs, le ou les capteurs étant configurés pour surveiller un état associé au ou aux éléments allongés (199) et/ou à la dent ou aux dents (1000) ; ou le dispositif comprend un ou plusieurs capteurs, le ou les capteurs étant configurés pour surveiller un état associé au ou aux éléments allongés (199) et/ou à la dent ou aux dents (1000) et le ou les capteurs sont configurés pour détecter la force exercée sur le ou les éléments allongés (199) et/ou sur la dent ou les dents (1000) et/ou la distance parcourue par le ou les éléments allongés (199) et/ou la dent ou les dents (1000).

8. Un dispositif dentaire (99) selon l'une quelconque des revendications précédentes, dans lequel : le processeur est compris dans un microcontrôleur.

9. Un dispositif dentaire (99) selon l'une quelconque des revendications précédentes, dans lequel le moyen de réduction et/ou de libération de la force est commandé par le processeur (6).

10. Un dispositif dentaire (99) selon l'une quelconque des revendications précédentes, comprenant en outre : un verrouillage d'élément allongé ; ou un verrouillage d'élément allongé, dans lequel le verrouillage d'élément allongé inclut un préhenseur d'élément allongé (4).

11. Un dispositif dentaire (99) selon l'une quelconque des revendications précédentes, dans lequel le moteur (5) est muni d'un mécanisme d'embrayage.

12. Appareil permettant de commander le déplacement d'une ou plusieurs dents comprenant un dispositif dentaire (99) selon l'une quelconque des revendications 1 à 11, un dispositif informatique programmé avec une application logicielle et un moyen de transmission de signaux fourni pour la transmission de signaux entre le dispositif informatique et le dispositif dentaire.
